# EUROPEAN PATENT SPECIFICATION

(11) **EP 2 611 422 B1**
(45) Date of publication and mention of the grant of the patent: **31.10.2018**
(21) Application number: 11755042.6
(22) Date of filing: 31.08.2011
(51) Int. Cl.: A61K 9/14, A61K 31/58, A61P 11/06, A61K 31/137

(54) **DRY POWDER INHALATION DRUG PRODUCTS EXHIBITING MOISTURE CONTROL PROPERTIES AND METHODS OF ADMINISTERING THE SAME**
INHALATIONSMEDIKAMENTE IN TROCKENPULVERFORM MIT FEUCHTIGKEITSREGELUNGSEIGENSCHAFTEN UND VERFAHREN ZU IHRER VERABREICHUNG
PRODUITS MÉDICAMENTEUX POUR INHALATION SOUS FORME DE POUDRE SÈCHE PRÉSENTANT PROPRIÉTÉS DE RÉGULATION D'HUMIDITÉ ET LEURS PROCÉDÉS D'ADMINISTRATION

(30) Priority: 31.08.2010 US 378409 P
(43) Date of publication of application: 10.07.2013
(73) Proprietor: GlaxoSmithKline Intellectual Property Development Limited, Brentford Middlesex TW8 9GS (GB)
(72) Inventor: ASWANIA, Osama, Stevenage Hertfordshire SG1 2NY (GB); JIANG, Zhong, Ware Hertfordshire SG12 0DP (GB); ROCHE, Trevor, Charles, Ware Hertfordshire SG12 0DP (GB); WHITAKER, Mark, Ware Hertfordshire SG12 0DP (GB)
(74) Representative: Florence, Julia Anne
(86) International application number: PCT/EP2011/065055
(87) International publication number: WO 2012/028662

(56) References cited:
- WO-A1-01/98174
- US-B2- 7 101 866

## Description

### FIELD OF THE INVENTION

The invention generally relates to inhalation drug products and methods of manufacturing the same.

### BACKGROUND OF THE INVENTION

Inhalers are hand-held portable devices that deliver medication directly to the lungs. One class of inhalers is passive dry powder inhalers ("DPI"). A passive DPI is a patient driven device wherein the action of breathing in through the device draws the powder formulation into the respiratory tract. DPI is well recognized as a method of drug delivery to the lung for treatment of pulmonary and systemic diseases.

A DPI formulation is generally a powder blend of active ingredients and a bulk solid diluent, such as lactose. The inhaled particle size of the active ingredients should be optimized to deliver the drug deep into the lung to achieve efficacy. This efficacious particle size, or fine particle mass ("FPM"), typically lies between 1-5 µm whereas particles larger than this, 5-10 µm, tend to be deposited in the upper airways without reaching the site of action and the very smallest particles, or very fine particle mass ("vFPM"), <1 µm, can resultantly be exhaled therefore typically not achieving the desired therapeutic levels. The Food and Drug Administration has recognized that the FPM of particles in a DPI device can be affected by environmental conditions, humidity in particular, and has suggested that manufacturers of such devices assess the effect of different environmental conditions on various interactive forces within the DPI device, which together influence the fluidization and aerosolization behavior of the formulation and, hence, performance (see FDA Guidance, Metered Dose Inhaler (MDI) and Dry Powder Inhaler (DPI) Drug Products Chemistry, Manufacturing, and Controls Documentation).

It is desirable to control the humidity within a DPI device, and hence the FPM. One approach to this involves the use of a desiccant system within the DPI such as shown in publication WO2008040841 (Lab Pharma Ltd., filed September 12, 2007). This approach uses a desiccant system comprising an air-tight container containing a dry desiccant, and a drug chamber containing the inhalation powder where the air-tight container is arranged inside the drug chamber or in its vicinity with the desiccant being capable of maintaining a fixed point of humidity as a saturated solution of at least one salt. The system is intended to maintain the maximum relative humidity around the powder formulation within a specified range for a prolonged period. Similarly, the reservoir based multi dose dry powder inhalers (e.g., Turbuhaler® made commercially available by Astra Zeneca of Wilmington, Delaware (see e.g., Wetterlim (Pharm. Res 5, 506-508, 1988)) contain a desiccant store in such inhalers.

An alternative approach to controlling the moisture absorption by dry powder products is shown in publication US20080063719 (Vectura Limited, filed April 29, 2005). This approach shows an inhalable dry powder formulation of glycopyrrolate with a stability of at least 1 year under normal conditions by storing in packaging made from a material which itself has a moisture content less than 10%, preferably less than 5% and more preferably less than 3%. Glycopyrrolate has been found to have an acute problem with respect to stability, especially immediately following conventional micronisation process. Individual capsule doses of glycopyrrolate for a suitable dispensing technique are used with the capsules being made of hypromellose, plasticized gelatin, starch, chitosan, synthetic plastic or thermoplastics. These materials were selected as capsules as they can maintain the glycopyrrolate with a suitable range of moisture for aerosol delivery.

Notwithstanding any potential progress that has been made regarding controlling the humidity within the DPI device, the range of acceptable humidity for specific actives and blends is not well understood and is difficult, if not impossible, to predict.

### SUMMARY OF THE INVENTION

In a first aspect, the invention provides a drug product. The drug product comprises a dry powder inhalation device having a pharmaceutical composition present therein, the pharmaceutical composition comprising a compound which is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; a hygroscopic material, and a package which encompasses the dry powder inhalation device and hygroscopic material defining an enclosed volume therein, wherein said hygroscopic material is in a state of partial hydration and wherein the enclosed volume within the package exhibits a Relative Humidity of from 20% to 40%.

By virtue of the invention, and more particularly by judicious selection of Relative Humidity values, and as set forth in greater detail herein, advantageously the drug product is capable of exhibiting improved shelf life and more stable fine particle mass as a result of the Relative Humidity of the package enclosed volume being controlled within a specified range..

In another aspect, the invention provides a method of treating a respiratory disorder. The method comprises administering to a patient by oral inhalation a compound which is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof using a drug product as described herein in the first aspect.

In another aspect, the invention provides a process of producing a drug product, comprising subjecting a hygroscopic material to moisture exposure sufficient to attain a predetermined relative humidity; combining the hygroscopic material with an inhalation device containing therein a pharmaceutical composition comprising a compound which is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof, and thereafter enclosing the dry powder inhalation device and hygroscopic material within a package to define an enclosed volume therein forming a drug product; wherein the hydration level of the hygroscopic material is such that the enclosed volume has a Relative Humidity of from 20% to 40%. The subjecting and combining steps may occur together or separately.

These and other aspects are provided by the invention as described herein.

### BRIEF DESCRIPTION OF THE FIGURES

Figure 1 depicts the % Nominal fine particle mass as a function of time for an inhaled formulation.
Figure 2 depicts the % Nominal fine particle mass as a function of time for an inhaled formulation.
Figure 3 depicts the % Nominal fine particle mass as a function of time for an inhaled formulation.
Figure 4 depicts the % Nominal fine particle mass as a function of time for an inhaled formulation.
Figure 5 depicts the % Nominal fine particle mass as a function of time for an inhaled formulation.
Figure 6 depicts the % Nominal fine particle mass as a function of time for an inhaled formulation.
Figure 7 depicts the % Nominal fine particle mass as a function of time for an inhaled formulation.
Figure 8 depicts the % Nominal fine particle mass as a function of time for an inhaled formulation.
Figure 9 depicts the mass median aerodynamic diameter as a function of time for an inhaled formulation.
Figure 10 depicts the mass median aerodynamic diameter as a function of time in an inhaled formulation.
Figure 11 depicts the mass median aerodynamic diameter as a function of time in an inhaled formulation.
Figure 12 depicts the mass median aerodynamic diameter as a function of time in an inhaled formulation.
Figure 13 depicts the mass median aerodynamic diameter as a function of time in an inhaled formulation.
Figure 14 depicts the geometric standard deviation as a function of time for an inhaled formulation.
Figure 15 depicts the geometric standard deviation as a function of time for an inhaled formulation.
Figure 16 depicts the geometric standard deviation as a function of time for an inhaled formulation.
Figure 17 depicts the geometric standard deviation as a function of time for an inhaled formulation.
Figure 18 depicts the geometric standard deviation as a function of time for an inhaled formulation.
Figure 19 represents an exploded view of an embodiment of a drug product in accordance with the invention.
Figure 20 represents an embodiment of a drug product in accordance with the invention.

### DETAILED DESCRIPTION OF THE INVENTION

The invention will now be described with respect to the embodiments presented herein. Before describing the present invention in detail, it is to be understood that this invention is not limited to particularly exemplified structures, apparatus, systems, materials or methods as such may, of course, vary. Thus, although a number of apparatus, systems and methods similar or equivalent to those described herein can be used in the practice of the present invention, the preferred apparatus, systems and methods are described herein.

It is also to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

Further, all publications, patents and patent applications cited herein, whether supra or infra, are hereby incorporated by reference in their entirety.

Finally, as used in this specification and the appended claims, the singular forms "a", "an", "the" and "one" include plural referents unless the content clearly dictates otherwise. Thus, for example, reference to "a salt" includes two or more such salts; reference to "a constituent" includes two or more such constituents and the like.

In one aspect, the invention provides drug product. The drug product comprises a dry powder inhalation device having a pharmaceutical composition present therein, the pharmaceutical composition comprising a compound which is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; a hygroscopic material, and a package which encompasses the dry powder inhalation device and the hygroscopic material defining an enclosed volume therein, wherein said hygroscopic material is in a state of partial hydration and wherein the enclosed volume within the package exhibits a Relative Humidity of from 20% to 40%.

Notwithstanding this range, in various embodiments, the Relative Humidity in the enclosed volume may range, at a lower end, from any of the values 20, 21, 22, 23, 24, 25, 26, 27, 28, 29, 30, 31, 32, 33, 34, 35, 36, 37, 38, 39 or 40 to, at an upper end, of any of the values, 20, 21, 22, 23, 24, 25, 26, 27, 28, 29, 30, 31, 32, 33, 34, 35, 36, 37, 38, 39, or 40%. In addition to ranges, the invention includes all singular values mentioned above.

Such relative humidity is obtained by applying suitable conditions to appropriate structures as described herein.

For the purposes of the invention, the term "pharmaceutical composition" may encompass pharmaceutical formulations and in particular, dry powder pharmaceutical formulations suitable for administration via inhalation.

The term "hygroscopic material" may encompass, without limitation, desiccants and humectants. Used herein, a hygroscopic material is one having the property of readily imbibing moisture from the atmosphere, while a desiccant is a drying agent, a substance which absorbs moisture and a humectant is any substance that is added to another substance to keep it moist.

In various embodiments, the pharmaceutical composition comprises the compound. In various embodiments, the pharmaceutical composition may consist essentially of the compound, i.e., exclude other active ingredients or medicaments. In various embodiments, the pharmaceutical composition may consist of the compound and at least one inert ingredient, as described herein, or the compound without inert ingredients.

The term "drug product" is to be construed to encompass dry powder inhalation device, hygroscopic material and a package which encloses the dry powder inhalation device and the hygroscopic material. In a preferred embodiment, the package is a low-moisture permeable package. As an example, the term "low-moisture-permeable-package may be defined to have a low moisture vapor transmission rate (MVTR) that is believed to be dependent on the amount of hygroscopic material (e.g., desiccant) used in the drug product. As an example, assuming that 8 gms as a maximum amount of hygroscopic material is used, then the MVTR should not exceed 2 mg/day/drug product, measured according to standard technique.

Hygroscopic materials, which encompass, for example, desiccants and humectants, can be pre- or partially hydrated to reach a predetermined level of moisture absorption or adsorption. These techniques are typically based on weight gain wherein the dry hygroscopic material is brought up to a percent absorbed or adsorbed moisture designed to produce the desired %RH within the final product. This process of pre- or partial hydration can be accomplished via various embodiments. One embodiment would be exposing the desiccant to a predetermined temperature and humidity environment based on anticipated storage conditions, for example, 25°C and 20%RH for a time up to the equilibrium point i.e. the time when weight gain due to moisture absorption stops increasing. Another embodiment would be an accelerated hydration process in which a chamber with higher than normal humidity level is used to shorten the time of exposure to pre-hydrate the desiccant material, for example, 25°C and 80 %RH for a 12 to 18 hour period.

Another way to reach the same predetermined moisture content level is by saturating the hygroscopic material first with water and then controlled drying it to the desired level of moisture content designed to produce the desired %RH within the final product. Any of the hydration techniques can be accomplished with vapor or liquid water.

The composition comprises a compound which is (6α,11β,16α,17α)-6,9-difluoro-17-{[(fluoromethyl)thio]carbonyl}-11-hydroxy-16-methyl-3-oxoandrosta-1,4-dien-17-yl 2-furancarboxylate or a solvate thereof, described in U.S. Patent No. 7,101,866, along with methods of making the same, the disclosure of which is incorporated by reference herein in its entirety. Such compounds are antiinflammatory compounds of the androstane series. Solvates of this compound which are suitable for use in medicine are those wherein the counterion or associated solvent is pharmaceutically acceptable, and are also within the scope of the invention. However, solvates having non-pharmaceutically acceptable counterions or associated solvents may be suitable.

The compound may be represented by the following formula:

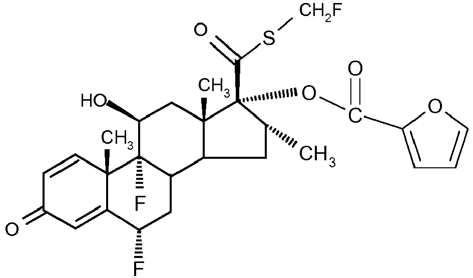

The above compound is known as fluticasone furoate.

The compositions (e.g., formulations) may be prepared according to methods that are known in the art, as well as components (e.g., inert ingredients) that make up the same. Along with the compound described herein, such formulations may also include at least one inert ingredient. Inert ingredients are broadly defined to include excipients, carriers, additives that improve stability performance, and the like.

Examples of excipients include mono-saccharides, such as mannitol, arabinose, xylitol and dextrose and monohydrates thereof, disaccharides, such as lactose, maltose and sucrose, and polysaccharides such as starches, dextrins or dextrans. More preferred excipients comprise particulate crystalline sugars such as glucose, fructose, mannitol, sucrose and lactose. Especially preferred excipients are anhydrous lactose and lactose monohydrate.

Generally, the particle size of the excipient particles will be much greater than that of the compound and as a result, do not penetrate into the respiratory tract. Thus, excipient particles for inhalable compositions may typically have particle sizes greater than 20µm, more preferably in the range 20 - 150µm. If desired, the inhalable compositions may also contain two or more excipient particle size ranges. For example, in one embodiment, in order to control the proportion of inhaled medicament, while retaining a good accuracy for metering, it may be desirable to use one component of the excipient that has a particle size of less than 15µm (the fine excipient component) and another component of the excipient that has a particle size of greater than 20µm but lower than 150µm, preferably lower than 80µm (the coarse excipient component).

The proportion of excipient material to be used in the inhalable compositions of this invention (e.g., lactose) may vary depending upon the proportion of each active agent, the powder inhaler for administration etc. The proportion may, for example, be about 75% to 99.5% by weight of the composition as a whole. In other embodiments, the excipient material may range from 94 to 99%, e.g. 97.7 to 99.0% by weight of the composition as a whole. In addition, the dry powder pharmaceutical formulation may also include an additive which improves stability performance, e.g. magnesium stearate or calcium stearate. Where such additives are present, in one embodiment, their concentration may range from 0.1% w/w to 2.0% w/w. In various embodiments, their concentration may range of 0.2 to 2%, e.g. 0.6 to 2%%, e.g. 0.75%, 1%, or e.g., 1.25% or 1.5% w/w. In one embodiment, the magnesium stearate will typically have a particle size in the range 1 to 50µm, and more particularly 1 - 20µm, e.g.1-10µm.

The dry powder formulations in accordance with the present invention can be prepared according to standard methods. As an example, pharmaceutically active agent or agents and inert ingredient can be intimately mixed using any suitable blending apparatus, such as high shear blenders. The progress of the blending process can be monitored by carrying out content uniformity determinations. For example, the blending apparatus may be stopped, materials removed using a sample thief and then analyzed for homogeneity by High Performance Liquid Chromatography (HPLC).

The dry powder compositions for use in accordance with the present invention are administered via inhalation devices. As an example, such devices can encompass capsules and cartridges of for example gelatin, or blisters of, for example, laminated aluminum foil. In various embodiments, each capsule, cartridge or blister may contain doses of composition according to the teachings presented herein. Examples of inhalation devices can include those intended for unit dose or multi-dose delivery of composition, including all of the devices set forth herein. As an example, in the case of multi-dose delivery, the formulation can be pre-metered (e.g., as in Diskus®, see GB2242134, U.S. Patent Nos. 6,032,666, 5,860,419, 5,873,360, 5,590,645, 6,378,519 and 6,536,427 or Diskhaler, see GB 2178965, 2129691 and 2169265, US Pat. Nos. 4,778,054, 4,811,731, 5,035,237) or metered in use (e.g. as in Turbuhaler, see EP 69715, or in the devices described in U.S. Patent No 6,321,747). An example of a unit-dose device is Rotahaler (see GB 2064336). In one embodiment, the Diskus® inhalation device comprises an elongate strip formed from a base sheet having a plurality of recesses spaced along its length and a lid sheet peelably sealed thereto to define a plurality of containers, each container having therein an inhalable formulation containing the compound optionally with other excipients and additive taught herein. The peelable seal is an engineered seal, and in one embodiment the engineered seal is a hermetic seal. Preferably, the strip is sufficiently flexible to be wound into a roll. The lid sheet and base sheet will preferably have leading end portions which are not sealed to one another and at least one of the leading end portions is constructed to be attached to a winding means. Also, preferably the engineered seal between the base and lid sheets extends over their whole width. The lid sheet may preferably be peeled from the base sheet in a longitudinal direction from a first end of the base sheet.

A dry powder composition may also be presented in an inhalation device which permits separate containment of two different components of the composition. Thus, for example, these components are administrable simultaneously but are stored separately, e.g. in separate pharmaceutical compositions, for example as described in WO 03/061743 A1 WO 2007/012871 A1 and/or WO2007/068896. In one embodiment an inhalation device permitting separate containment of components is an inhaler device having two peelable blister strips, each strip containing pre-metered doses in blister pockets arranged along its length, e.g., multiple containers within each blister strip. Said device has an internal indexing mechanism which, each time the device is actuated, peels opens a pocket of each strip and positions the blisters so that each newly exposed dose of each strip is adjacent to the manifold which communicates with the mouthpiece of the device. When the patient inhales at the mouthpiece, each dose is simultaneously drawn out of its associated pocket into the manifold and entrained via the mouthpiece into the patient's respiratory tract. A further device that permits separate containment of different components is DUOHALER™ of Innovata. In addition, various structures of inhalation devices provide for the sequential or separate delivery of the pharmaceutical composition(s) from the device, in addition to simultaneous delivery.

In another aspect, the invention includes a drug product for use in treating respiratory disorders. This includes administering to a subject in need thereof of, a therapeutically effective amount of a dry powder pharmaceutical composition as described herein above, comprising a compound which is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof, and at least one excipient using the drug product of the first aspect of the invention.

The term "treatment" is to be construed as encompassing the amelioration or management of, without limitation, any of the respiratory disorders set forth herein, wherein treatment may be construed to include prophylaxis. Examples of respiratory disorders include diseases associated with reversible airways obstruction such as asthma, chronic obstructive pulmonary diseases (COPD) (e.g. chronic and wheezy bronchitis, emphysema), respiratory tract infection and upper respiratory tract disease (e.g., rhinitis, including seasonal and allergic rhinitis).

The compound administered according to the method described herein above may be administered once-daily, or multiple times per day (e.g., twice, three-times, etc). In one embodiment, as an example, the dose per administration for the compound may range from 25µg to 800µg. In one embodiment, the compound may be administered by inhalation at a dose of from 25µg to 800µg daily, and if necessary in divided doses. Thus, in various embodiments, the daily dose of compound may be for example 25, 50, 100, 200, 300, 400, 600 or 800 µg.

In a preferred embodiment, an inhalation device as taught by WO 03/061743 A1 WO 2007/012871 A1 and/or WO2007/068896 is used containing strips of 30, 14 or 7 regularly distributed blisters. For various embodiments, examples of three compositions in such a device may be as follows below. In every instance, such compositions are contained within blisters of a single strip, while the other strip is void.

### Composition I:

Compound: 50mcg (micronized), lactose monohydrate: to 12.5mg

### Composition II:

Compound A: 100mcg (micronized), lactose monohydrate: to 12.5mg

### Composition III:

Compound A: 200mcg (micronized), lactose monohydrate: to 12.5mg

In various embodiments, the drug product of the present invention may include a package for storage of the inhalation device described herein, and the package may be formed from a material capable of controlling the ingress of moisture thereto or egress or moisture therefrom. Such a package for storage of a container is often referred to as an overwrap. In various embodiments, the drug products may be free of overwraps. Examples of such packages are described in WO 01/98174. In one embodiment, the package may be impermeable or substantially impermeable to moisture (i.e., less than 0.1mg/day). In another embodiment, the package may control the ingress or egress of moisture such that the ambient moisture content within the package is essentially constant, such as varying by no more than ±20% RH, preferably by less than ±10% RH over a 2 year shelf life. Ambient moisture content may for example be measured as the relative humidity within the package. In another embodiment, the package may enable moisture transfer in one way only i.e. ingress only or egress only. In another embodiment, the package may enable moisture transfer to either a set minimum /maximum moisture content within the package or within a set minimum / maximum moisture transfer rate.

In one embodiment, the package may be wrappable and sealable around the inhalation device to form an enclosed volume in which the device is present. Such techniques of wrapping and sealing are known in the art. As an example, the sealing comprises heat sealing said packaging material. In other aspects, the seal is formed by ultrasonic welding, heat stamping, adhesive or laser welding methods.

In a further embodiment, the package may have a preformed tray into which the inhalation device is placed and a subsequent sealing lid which is affixed thus forming an enclosed volume in which the device is present. Such techniques of preforming and sealing are known in the art. As an example, the preformed tray is an aluminum and polypropylene laminate and the lid is also an aluminum and polymer laminate. Embodiments of the laminate include, without limitation, those of 110-160 micron aluminum and 30 micron polypropylene, and 60 micron aluminum and 25 g/m² polymer. Preferably, the preformed tray and lid should be of sufficient size to accommodate an inhalation device and desiccant sachet, e.g., a sachet of 70 x 50 x 5 mm. An example of a commercial preformed tray and lid is one provided by Amcor of Bristol, United Kingdom. Such an embodiment may be free of an overwrap material.

A preferred embodiment of a preformed tray and lid is provided by Constantia of Weinburg, Austria. With respect to the tray, and in one preferred embodiment, materials and tolerances are as follows (from outside to inside):

| | |
|---|---|
| Lubricant | 0.7 g/m² |
| Lacquer | 2.5 g/m². |
| Aluminum | 0.11-0.16mm |
| Adhesive | 6 g/m² |
| Polypropylene, | 0.030 mm |
| Peel force of 15 mm wide sample when sealed to reference lid laminate | 16.5 N |

With respect to the lid, in one or more embodiments, approved materials and tolerances are as follows (from outside to inside):

| | |
|---|---|
| Lacquer | 0.8 g/m² |
| Aluminum | 0.060 mm |
| Polypropylene based co-extrusion coating | 25 g/m² |
| Total thicknesses including embossing by micrometer or equivalent | 135 µg |
| | |
| Peel force of 15 mm wide sample when sealed to reference lid laminate | 16.5 N |

In a preferred embodiment, when the lid and tray are sealed, they are capable of protecting the dry powder inhalation device from excessive humidity throughout its shelf-life, e.g., the package may have an MVTR of 0.55mg/24hr/pack at 30°C. When the package is sealed, it is preferred that the lid and tray should be capable of withstanding a partial vacuum of 14,000 ft equivalent. In a preferred embodiment, when sealed, the tray/lid combination should provide an opening peel force that is no greater than 20N across the entire opening to allow for relative ease of patient opening.

With respect a preferred embodiment of the lid/tray laminate, in regards to the tray, an aluminum layer of 110 microns is considered as a baseline. It is preferred that the lid be an aluminum/polypropylene laminate and that an aluminum layer of 60 microns be considered baseline.

In accordance with the invention, the package for storage includes a hygroscopic material. The hygroscopic material may be incorporated into the product in a number of ways. As an example, in one embodiment, the package includes a hygroscopic material within the enclosed volume. In an embodiment, the inhalation device and the hygroscopic material are sealable within the overwrap. In one embodiment, the hygroscopic material may be included as a free-moving or free-flowing sachet within the drug product. Examples of partially hydrated hygroscopic materials include, without limitation, those selected from the group consisting of partially hydrated forms of silica gel, zeolite, alumina, bauxite, calcium oxide activated bentonite clay, water-absorbing clay, molecular sieve and any mixtures thereof. Such partially hydrated forms can provide relative humidity within the product pack within specified range. In another embodiment, the overwrap or inhaler device may be lined, coated or impregnated with hygroscopic material. Examples of humectants include, without limitation, glycerol, sorbitol, polyethylene glycol, mono- and oligomeric sugars, sodium pyroglutamate, sodium tripolyphosphate, monopotassium phosphate, lactic acid and urea. Examples of desiccants include, without limitation, montmorillonite clay, silica gel, crystalline aluminosilicates, calcium oxide, calcium sulfate, activated alumina, metal salts and phosphorus compounds.
The overwrap may be present in the form of flexible packaging material. In various embodiments, the flexible packaging material can be any material which is impervious to or substantially impervious to moisture. The overwrap utilizing such material is typically present as a laminate structure. In one embodiment, the flexible packaging material preferably comprises a non-thermoplastic substrate (such as, for example, a metal foil, e.g. aluminum, of 9µm thickness) and a heat sealable layer disposed thereon, and an additional protective layer, such as, for example, a polymer film of polyester. To further reduce moisture ingress, thicker metal films, such as 12 µm to 20 µm, may be used. In various embodiments, the heat sealable layer is usually disposed on the inner surface of the assembled package. In various embodiments, the additional protective layer is usually disposed on the surface opposite the heat sealable layer. In various embodiments, the RH may be measured within the overwrap.

The substrate is preferably formed from aluminum foil. However, other metals for the substrate include, but are not limited to, tin, silver, iron, zinc, or magnesium formed on a sheet by vacuum deposition or sputtering and a carboxyl group-containing polyolefin layer formed on the metal layer by lamination.

The heat sealable layer can be formed from any thermoplastic or thermosetting material such as an ionomer resin, polyolefin, or cycloolefin copolymer. Ionomer resins typically include ionically cross- linked ethylene-methacrylic acid and ethylene acrylic acid copolymers. Properties which distinguish these ionomers resins from other polyolefin heat-sealed polymers are high clarity, high impact resistance, low haze in lamination, tear resistance, abrasion resistance, solid state toughness, and moisture imperviousness. In an embodiment, the heat sealable layer is made out of SURLYN™ (an ionomer resin) or a form of polyethylene to provide sufficient heat sealing properties.

The outer protective layer, if present, can be formed of any material as long as the final laminate has the requisite properties. In one embodiment, as an example, the protective layer (e.g., polyester) is adhesively laminated to the substrate (e.g., aluminum) and the substrate layer in turn is adhesively laminated to the heat sealable layer (e.g., the ionomer film or SURLYN™ (an ionomer resin).

Examples of thicknesses of the three layers include a protective layer of 20 µm to 30 µm; and a substrate layer of 9 µm to 20 µm. For the heat sealable layer, examples of thicknesses range from 40 to 70 µm.

Adhesives may be used to join the respective layers of materials together. The adhesive layers are typically substantially smaller in thickness relative to the thickness of the substrate, heat sealable and/or protective layers which they bond.

Specific embodiments of overwrap materials are as follows:
1) polyethylene terephthalate ("PET") 12µm (17gsm)/Extr/aluminum 9µm (24gsm)/Extr/low density polyethylene ("LDPE") 35µm 32gsm)
2) PET 12µm (17gsm)/adhesive 4 gsm/aluminum 12µm (32 gsm)/adhesive 3 gsm/LDPE without additives 50 µm (46 gsm)

The hygroscopic materials utilize moisture absorbing materials, such as desiccants and humectants, and a moisture absorbing material is preferably present in the form of a silica gel desiccant sachet. However, other vapor or moisture absorbing mechanisms are not beyond the scope of the present invention. Other vapor or moisture absorbing materials include desiccants and humectants made from inorganic materials such a zeolites and aluminas. Such inorganic vapor or moisture absorbing materials have high water absorption capacities and favorable water absorption isotherm shapes. The water absorption capacity of such materials typically varies from 20 to 50 weight percent and the percentage moisture content which controls the relative humidity inside the product pack within a specified range typically varies from 3 to 20 weight percent. Silica gel is particularly suited for enabling hydration between, at a lower end, from any of the values 15, 16, 17, 18 19, 20, 21, 22, 23, 24, 25, 26, 27, 28, 29, 30, 31, 32, 33, 34, 35, 36, 37, 38, 39 or 40% RH to, at an upper end, of any of the values, 17, 18, 19, 20, 21, 22, 23, 24, 25, 26, 27, 28, 29, 30, 31, 32, 33, 34, 35, 36, 37, 38, 39, or 40% RH. %RH as silica gel is capable of adsorbing up to 35% of its own weight in moisture (preferably up to 30%) and thus is capable of maintaining %RH levels up to 50%. Preferred silica gel sachet can be present as Eq-Pak® fan folded sachets or Eq-Pak® individually cut sachets made commercially available by Sud-Chemie of France. In a preferred embodiment, the absorbing material is present inside TYVEK®, which is a nylon mesh bonded with HDPE fibers, and made commercially available by E.I. Dupont de Nemours of Wilmington, Delaware.

In various embodiments, the term "partial hydration" refers to a hygroscopic material being less than fully saturated. The hygroscopic material (e.g., silica gel) may have a moisture content range of from , at a lower end, any of the values, 8.4, 8.8, 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6% w/w to, at a higher end, any of the values, 9.2, 9.6, 10, 10.4, 10.8, 11.2, 11.6, 12, 12.5, 12.9, 13.3, 13.7, 14.1, 14.6, 15, 15.4, 15.8, 16.2, 16.6, 17, 17.4, 17.8, 18.2, or 18.6%, In addition to ranges, such embodiments include all singular values mentioned above, i.e., the moisture content may be 8.4, 8.8, 9.2% , etc, through 18.6%. w/w. The above values are w/w of total mass based on the material being completely dry at 0%w/w and fully saturated at around 30%-31% (wt. water/wt. hygroscopic material). In another embodiment, such range can be expressed as the Percentage Relative Humidity range of controlled pack, usually less than 500cc in volume, from, at a lower end, any of the values 15, 16, 17, 18 19, 20, 21, 22, 23, 24, 25, 26, 27, 28, 29, 30, 31, 32, 33, 34, 35, 36, 37, 38, 39 or 40 to, at an upper end, of any of the values, 17, 18, 19, 20, 21, 22, 23, 24, 25, 26, 27, 28, 29, 30, 31, 32, 33, 34, 35, 36, 37, 38, 39, or 40%RH, including all singular values within these ranges, as well as other ranges taught herein.

Preferred embodiments of desiccant sachets are 50x70mm TYVEK® bags containing 2 - 10 grams (e.g., 4 or 8 grams) of partially hydrated silica gel (Eq-pak®). The Eq-pak® sachets may provide from 15% to 40% RH within the product pack. These sachets may come in two forms: (1) fan-folded, which is of strip of connected sachets of approximately 1000 per bag and (2) individually-cut, which are supplied in bags of 100 each.

Specific embodiments of desiccants **A, B** and **C** are set forth in Tables 1-3:

**Table 1**

| | |
|---|---|
| **"A"** | Eq-Pak 30%RH |
| **Absorber Material** | Silica Gel |
| **Size/Capacity** | Net Weight: 8.0g (+/- 10%) of Silica Gel Relative Humidity in Packaging: 30%RH (+/-3%) @ 20°C(+/-3°C) |
| **Misc** | Residual Moisture Level: 15.8% w/w (+/-1%) |
| **Container Material (Desiccant Sachet)** | Tyvek® |
| **Dimensions** | Length: 70mm |
| | Width: 50mm |

**Table 2**

| | |
|---|---|
| **"B"** | Eq-Pak 20%RH , |
| **Absorber Material** | Silica Gel |
| **Size/Capacity** | Net Weight: 8.0g (+/- 10%) of Silica Gel Relative Humidity in Packaging: 20%RH (+/-3%) @ 20°C(+/-3°C) |
| **Misc** | Residual Moisture Level: 11.3% w/w (+/-1%) |
| **Container Material (Desiccant Sachet)** | Tyvek® |
| **Dimensions** | Length: 70mm |
| | Width: 50mm |

**Table 3**

| | |
|---|---|
| **"C"** | Eq-pak 10%RH |
| **Absorber Material** | Silica Gel |
| **Size/Capacity** | Net Weight: 8.0g (+/- 10%) of Silica Gel Relative Humidity in Packaging: 10%RH (+/-3%) @ 20°C(+/-3°C) |
| **Misc** | Residual Moisture Level: 6.8% w/w (+/-1%) |
| **Container Material (Desiccant Sachet)** | Tyvek® |
| **Dimensions** | Length: 70mm |
| | Width: 50mm |

The RH of desiccant sachets may be measured, for example, by sealing a desiccant sachet inside a small foil laminate pouch then inserting a pointed, narrow RH probe into the pouch and recording the RH when a stable value is attained (i.e., little or no variation), which as an example, typically occurs in two minutes. Typically a Rotonic Hygropalm meter with SC04 probe is used, although other measuring devices/techniques may be employed without departing from the scope of the invention.

Other exemplary moisture absorbing materials include, but are not limited to, alumina, bauxite, anhydrous, calcium sulphate, water-absorbing clay, activated bentonite clay, a zeolite molecular sieve, or other like materials which optionally include a moisture sensitive colour indicator such as cobalt chloride to indicate when the desiccant is no longer operable.

The desiccant should be present in an amount sufficient to absorb any excess moisture inside the package. When silica gel is used, and in one example, 1g to 12g of silica gel is sufficient for a typical dry powder inhaler. Moreover, the desiccant can be present in an amount sufficient to absorb any moisture that possibly ingresses from the external environment or that is present in the polymers used to fabricate the device. It is also possible to place the desiccant adjacent to the inhalation device. As an example, in embodiments in which an overwrap material envelops the inhalation device, the desiccant may be present therein in a loose or free-flowing manner. In other embodiments, the desiccant can be secured to the inside of the overwrap by structures know in the art, such as those disclosed in WO 01/98174.

In another aspect, the invention provides a process of producing a drug product. The process comprises subjecting a hygroscopic material to moisture exposure (e.g., water or humidity) sufficient to attain a predetermined moisture content and thereby a predetermined relative humidity (e.g., via partial hydration as described herein); combining the hygroscopic material with an inhalation device including therein a pharmaceutical composition comprising (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof and then enclosing the dry powder inhalation device and hygroscopic material within a package to define an enclosed volume therein, wherein the hydration level of the hygroscopic material is such that the enclosed volume has an %RH of from, 20 to 40% RH. Such moisture exposing conditions relating to predetermined relative moisture exposure are described herein. The invention also includes drug products made by such processes. The RH within the moisture protective container enclosing the drug product may be measured by inserting a pointed, narrow RH probe into the pouch and recording the RH when a stable value is attained. Typically a Rotronic Hygropalm meter with SC04 probe is used, although other measuring devices/techniques can be used.

To further elaborate on the above, the dry powder inhalation device and hygroscopic material are combined by being brought together in sufficient proximity such that they may be packaged using accepted techniques, e.g., in a preferred embodiment the device and hygroscopic material are placed in a tray and thereafter are enclosed by placement of a lid over the tray opening.

The present invention is highly advantageous. By virtue of the elements of the drug product, the dry powder composition may be capable of exhibiting a fine particle mass (FPM) that is relatively stable, e.g., in one embodiment may deviate no more than +/- 15% from the nominal value of 20% of nominal total drug content per blister (i.e. 17-23%) for the compound over the product shelf-life of 2 years when the product is stored at 50 to 60% RH and 20°C to 25°C, and in another embodiment, when the product is stored at 75% RH and 30°C.

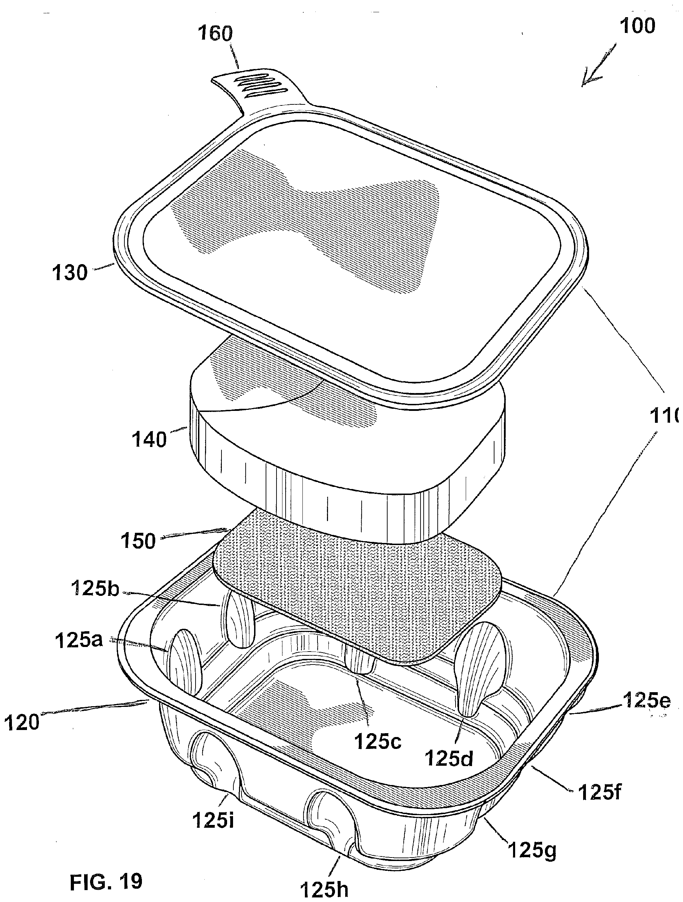

**FIG. 19** is a drawing representing a preferred embodiment of a drug product **100** according to the present invention. It should be appreciated that other embodiments are encompassed by the scope of the present invention and that this specific embodiment does not serve to limit that scope.

As shown, drug product **100** includes a package **110** in the form of a tray **120** and lid **130.** The tray **120** is conformed to receive a dry powder inhalation device **140** therein. Ribs **125a, 125b, 125c, 125d, 125e, 125f, 125g, 125h** and **125i** are present to minimize or prevent movement of device **140** therein. Also illustrated between device **140** and tray **120** is hygroscopic material **150.** In this embodiment, hygroscopic material **150** is present in the form of a loose desiccant packet. Lid **130** serves to enclose device **140** and hygroscopic material **150** within the tray **120** when sealed to the tray. As depicted, lid **130** contains a tab **160** to allow for relative ease of removal of the lid **130** from the tray **120.** Device **140** may thereafter be used in a conventional fashion.

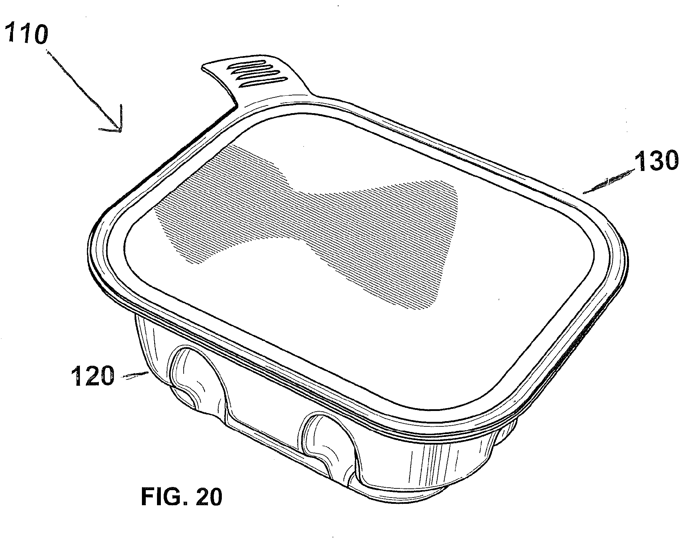

**FIG. 20** shows the lid **130** sealed to the tray **120** with device and hygroscopic material contained therein.

The invention will now be described with respect to the following examples. It should be appreciated that these examples are set forth for the purpose of illustrating the invention, and does not limit the scope of the invention as defined by the claims.

### Definition of terms

The following terms are used herein:
"Inhalation grade lactose" is comprised of alpha lactose monohydrate having a predetermined shape and particle size distribution. A source of inhalation grade lactose is Friesland Campina Domo in the Netherlands.

"Foil laminate 1" as used herein consists of a layered sheet of oriented polyester teraphthalate (12µm), aluminum foil (9µm), and low density polyethylene (35µm).

"Foil laminate 2" as used herein consists of a layered sheet of oriented polyester teraphthalate (12µm), adhesive (4gsm), aluminum foil (12µm), adhesive (3gsm), and low density polyethylene (50µm).

"Standard blending equipment" indicates that blending was performed using high shear blenders at a scale of 4-35kg. Examples of high shear blender are PMA and TRV high speed blender.

"Standard filling equipment" indicates that filling was performed using equipment described in U.S. Patent No. 5,187,921A or U.S. Publication No. 2005-0183395.

"Device" as used herein in the examples refers to inhalation device described in U.S. Publication No. 2008-0308102 A1.

"Standard stability chamber" are environmental chambers that can attain temperature accuracy and precision of +/- 2°C and a RH accuracy and precision of +/- 5%RH.

"Cascade impaction" refers to Apparatus 5 in *General Chapter <601> Aerosols, Nasal Sprays, MDIs & DPIs: Uniformity of Dosage Units* (MDI & DPI) USP30 used at 60l/min for a duration of 4 seconds per actuation. Typically 10-20 actuations are discharged per determination. "Fine Particle Mass" is defined as the sum of the deposition on stage 2 to stage 5 and is calculated as a percentage of the nominal drug target contained in each individual blister.

"HPLC" refers to standard high performance liquid chromatography.

"UV/visible detection" indicates an ultraviolet detector at the outlet of the HPLC.

"Fluorescence detection" indicates a fluorescence detector at the outlet of the HPLC.

"Lactose fines" are the proportion of the particle size distribution less than 15µm, measured by laser diffraction of a suspension (dry dispersion), or less than 4.5µm of an air dispersion (dry dispersion).

Compound "A" refers to (6α,11β,16α,17α)-6,9-difluoro-17-{[(fluoromethyl)thio]carbonyl}-11-hydroxy-16-methyl-3-oxoandrosta-1,4-dien-17-yl 2-furancarboxylate.

Compound "B" refers to 4-{(1*R*)-2-[(6-{2-[(2,6-dichlorobenzyl)oxy]ethoxy }hexyl)amino]-1-hydroxyethyl}-2-(hydroxymethyl)phenol triphenyl acetate. The quantities of Compound B quoted in the examples is for the free base. Graphical representation of the performance of Compound "B" throughout the examples can be found in the PCT International Patent Application, filed concurrently herewith, which claims priority to Serial No. 61/378,412 filed August 30, 2010, the disclosure of which is incorporated herein by reference in its entirety.

Unprotected product refers to a product unprotected from moisture and has been used interchangeable with the term 'naked'.

### Example 1

### Relationship of desiccant, humidity and temperature to inhalable formulation fine particle mass

Foil laminate 1 (overwrapped) and foil laminate 1 with a desiccant (overwrapped with desiccant) are employed for this example.

Blends were manufactured and filled using standard equipment. Blends were packaged in strips as individual actives and in devices as dual active products (i.e., one strip of each active). The blends consisted of four levels of active ingredient, 50µg and 800µg of Compound A and the balance to 12.5mg inhalation grade lactose (lactose fines 7 % and 4% < than 4.5 microns by dry dispersion respectively), and 100µg and 6.25µg of Compound B, 1% magnesium stearate, and the balance to 12.5mg of inhalation grade lactose (lactose fines 8% <15 microns by wet dispersion). Final packaged device drug contents were 6.25µg/50µg, 6.25µg /800µg, 100µg/50µg and 100µg /800µg of Compound B and Compound A, respectively (henceforth referred to as 6.25/50, 6.25/800, 100/50 and 100/800). Devices were overwrapped or overwrapped with a desiccant packet, using foil laminate 1. Desiccant consisted of an 8g 20% RH Eq-pak®. The relative humidity within the overwrapped packs without desiccant was around 45%RH. Overwrapped devices were subsequently stored in standard stability chambers and storage conditions were 25°C and 60% RH, 30°C and 75% RH and 40°C and 75% RH. Samples were analyzed for fine particle mass at time intervals up to 24 months for samples stored at 25°C and 60% RH and up to 6 months for samples stored at 30°C and 75% RH and 40°C and 75% RH. Fine particle mass was determined using cascade impaction with HPLC coupled to UV/visible and/or fluorescence detection.

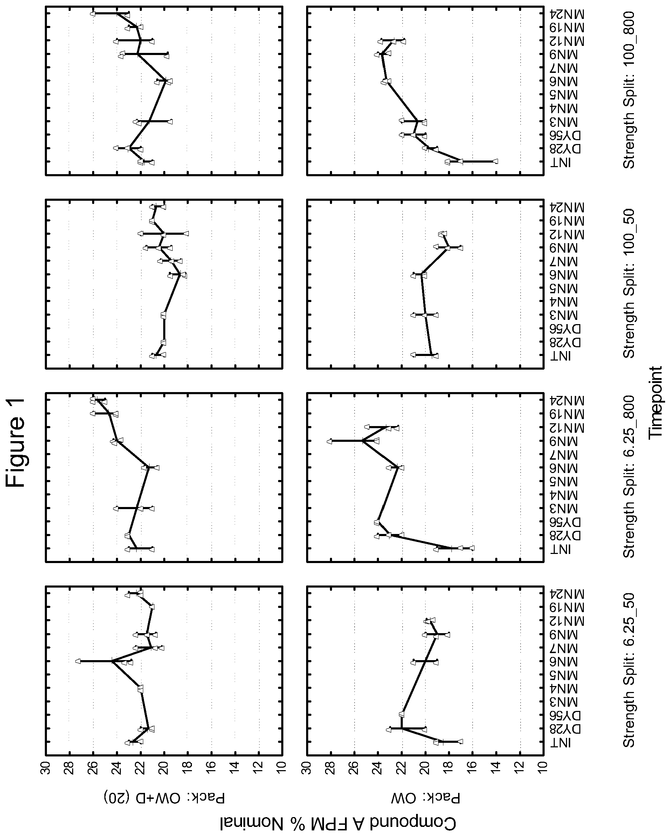

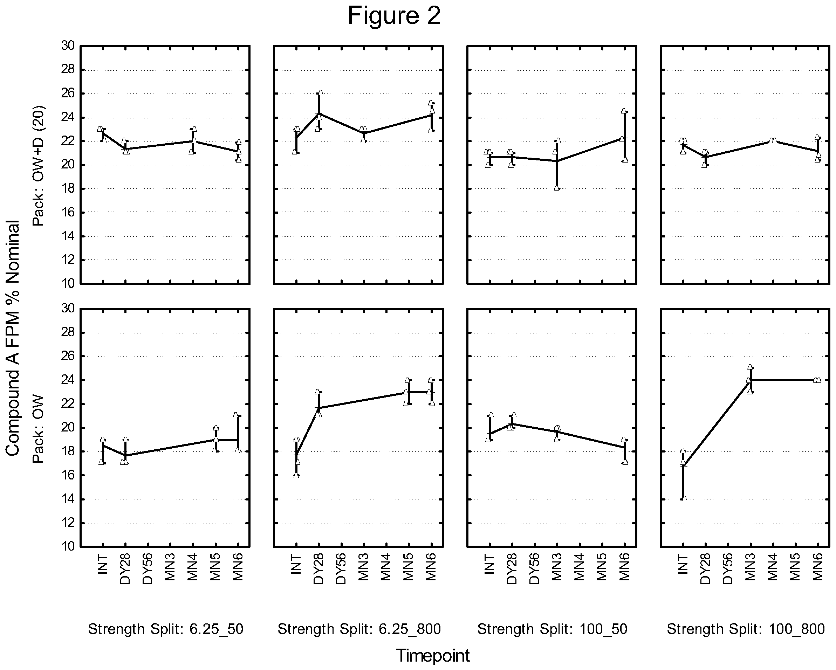

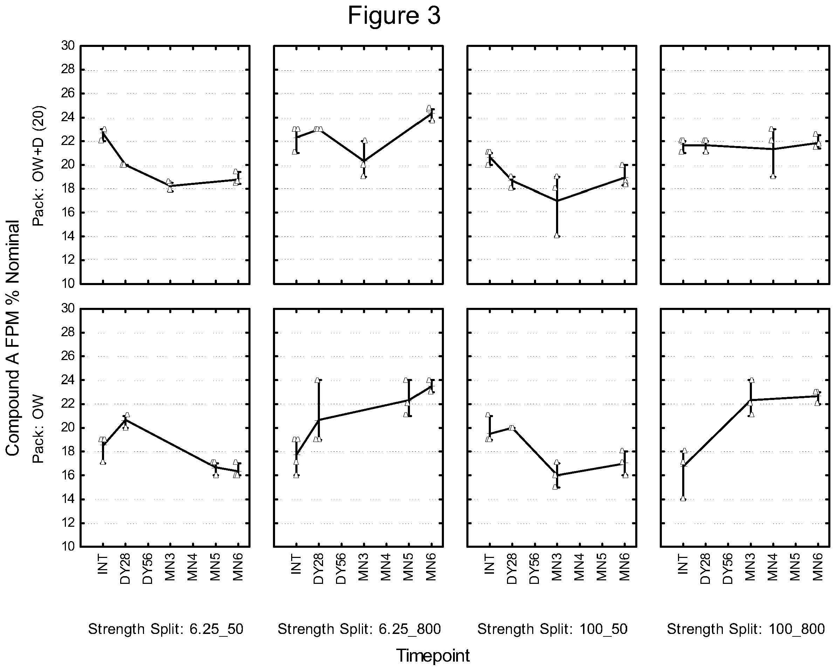

Figures 1-3 are illustrative of the changes in total fine particle mass as a percentage of the nominal drug per dose (%nominal) as a function of both desiccant and storage conditions at select time intervals for the 6.25/50, 6.25/800, 100/50 and 100/800 samples overwrapped (OW) and overwrapped with desiccant (OW+D). More specifically, Figures 1-3 show the changes in fine particle mass (% Nominal) for blends 6.25/50, 100/50, 6.25/800 and 100/800 for Compound A at 25°C/60% RH, 30°C/75% RH and 40°C/75% RH respectively.

### Example 2

### Relationship of desiccant, humidity and temperature to inhalable formulation fine particle mass

Blends were manufactured and filled using standard equipment. The inhalation device contained two packs in peelable blister strip form, the first containing blend with 12.5 µg of compound B, 1% magnesium stearate, and the balance to 12.5mg of inhalation grade lactose (lactose fines 4.5% < than 4.5 microns by dry dispersion) and the second peelable blister strip containing 50 µg of compound A and the balance to 12.5mg of inhalation grade (lactose fines 6.5% < than 4.5 microns by dry dispersion) lactose. Samples were left unprotected, overwrapped or overwrapped with a desiccant packet, using foil laminate 2. Desiccant humidity values were 15% (8g Eq-pak®, 10% RH plus 8g Eq-pak®, 20% RH), 20% (8g Eq-pak®, 20% RH)and 30% (8g Eq-pak®, 30% RH). The RH inside the OW packs was around 45% RH. Overwrapped samples and samples overwrapped with desiccant were subsequently stored in standard stability chambers and storage conditions were 25°C and 60% RH and 40°C and 75% RH. Unprotected samples were stored in standard stability chambers and storage conditions were 25°C and 75% RH. Samples were analyzed for fine particle mass initially and at time intervals up to 3 months for unprotected product stored at 25°C and 75%RH, up to 6 months for overwrapped and overwrapped with desiccant samples stored at 40°C and 75% RH and up to 9 or 12 months for overwrapped and overwrapped with desiccant samples stored at 25°C and 60% RH. Fine particle mass was determined using cascade impaction with HPLC coupled to UV/visible and fluorescence detection.

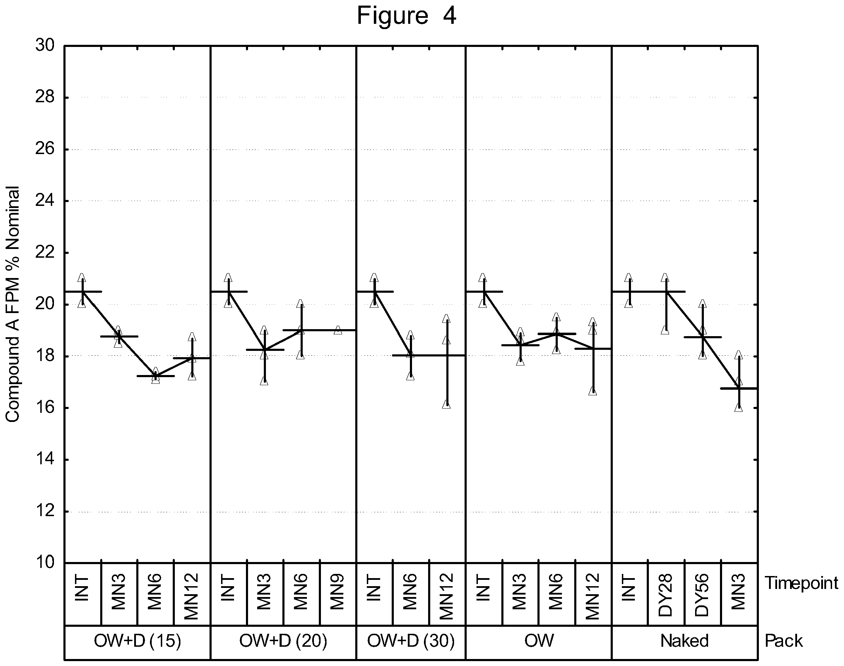

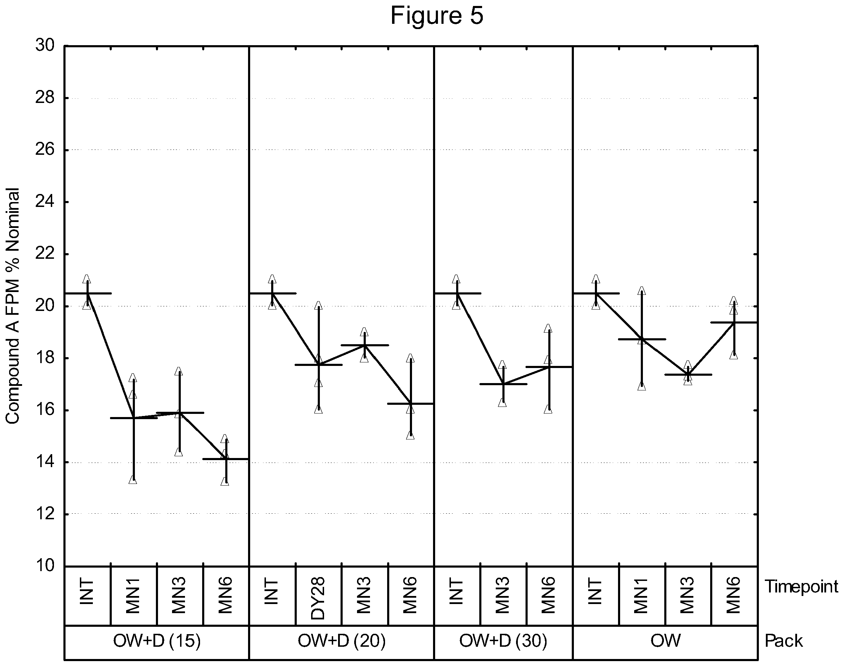

Figures 4-5 are illustrative of the changes in total fine particle mass as a percentage of the nominal drug per dose (%nominal) as a function of both desiccant RH values and storage time. Figure 4 shows fine particle mass values for samples stored overwrapped (OW) and overwrapped with desiccant (OW+D) at 25°C and unprotected at 25°C and 75%RH at select time intervals for compound A. Figure 5 shows fine particle mass values for samples stored overwrapped (OW) and overwrapped with desiccant (OW+D) at 40°C at select time intervals for compound A.

### Example 3

### Relationship of desiccant, humidity and temperature to inhalable formulation fine particle mass

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7 %, 6.2% and 5.7% < than 4.5 microns by dry dispersion), 25µg of Compound B, 1% magnesium stearate, and the balance to 12.5mg of inhalation grade lactose (lactose fines 4.5% < than 4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet (using foil laminate 1) or left unprotected. Desiccant consisted of an 10% (8g Eq-pak®, 10% RH), 15% (8g Eq-pak®, 10% RH plus 8g Eq-pak®, 20% RH), 20% (8g Eq-pak®, 20% RH), 30% (8g Eq-pak®, 30% RH) and 40% (8g Eq-pak®, 40% RH). Overwrapped devices were subsequently stored in standard stability chambers and storage conditions were 25°C and 60% RH and 40°C and 75% RH. Samples were analyzed for fine particle mass at time intervals up to 6 months for samples stored at 25°C and 60% RH and up to 7 months for samples stored at 40°C and 75% RH. Fine particle mass was determined using cascade impaction with HPLC coupled to UV/visible and/or fluorescence detection.

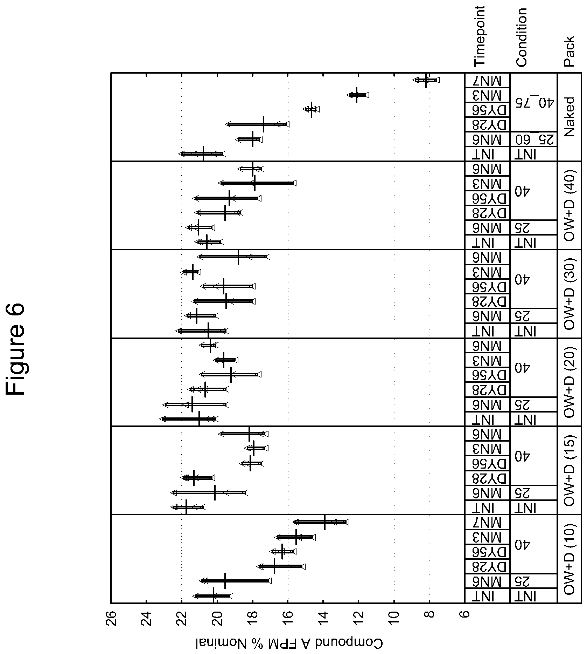

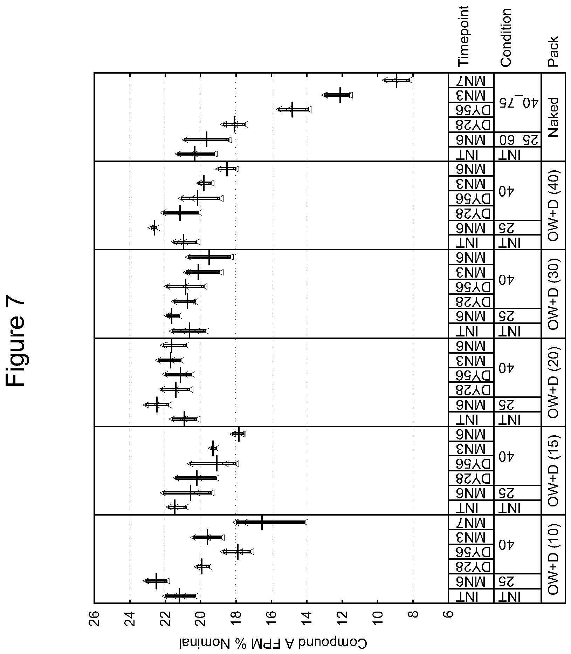

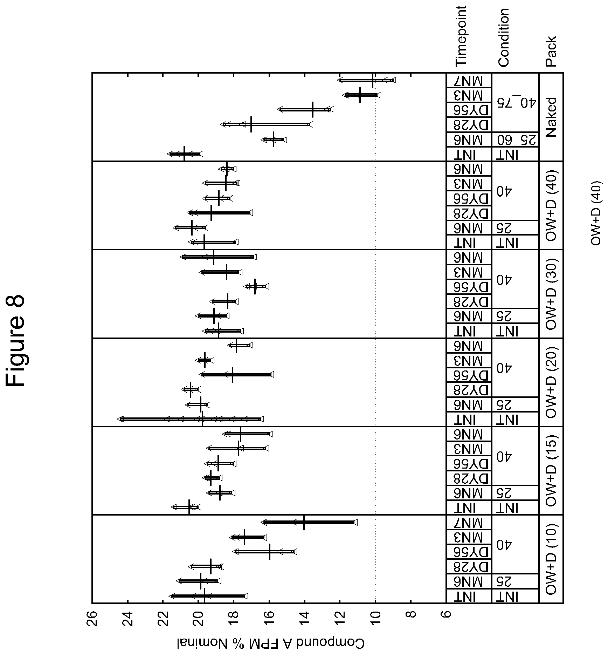

Figures 6-8 are illustrative of the changes in total fine particle mass as a percentage of the nominal drug per dose (%nominal) as a function of both desiccant and storage conditions at select time intervals for the 50/25, 100/25, and 200/25 samples overwrapped with desiccant (OW+D) and unprotected, and more specifically show the changes in fine particle mass (% Nominal) for blends 50/25, 100/25 and 200/25 for Compound A at 25°C/60% and 40°C/75% RH respectively.

### Example 4

### Relationship of desiccant, humidity and temperature to inhalable formulation mass median aerodynamic diameter (MMAD)

Blends were manufactured and filled using standard equipment. The inhalation device contained two packs in peelable blister strip form, the first containing blend with 12.5 µg of compound B, 1% magnesium stearate, and the balance to 12.5mg of inhalation grade lactose (lactose fines 4.5% < than 4.5 microns by dry dispersion) and the second peelable blister strip containing 50 µg of compound A and the balance to 12.5mg of inhalation grade (lactose fines 6.5% < than 4.5 microns by dry dispersion) lactose. Samples were left unprotected, overwrapped or overwrapped with a desiccant packet, using foil laminate 2. Desiccant humidity values were 15% (8g Eq-pak®, 10% RH plus 8g Eq-pak®, 20% RH), 20% (8g Eq-pak®, 20% RH) and 30% (8g Eq-pak®, 30% RH). The RH inside the OW packs was around 45% RH. Overwrapped samples and samples overwrapped with desiccant were subsequently stored in standard stability chambers and storage conditions were 25°C and 60% RH and 40°C and 75% RH. Unprotected samples were stored in standard stability chambers and storage conditions were 25°C and 75% RH. Samples were analyzed for MMAD initially and at time intervals up to 3 months for unprotected product stored at 25°C and 75%RH, up to 6 months for overwrapped and overwrapped with desiccant samples stored at 40°C and 75% RH and up to 9 or 12 months for overwrapped and overwrapped with desiccant samples stored at 25°C and 60% RH. MMAD was determined using cascade impaction with HPLC coupled to UV/visible and fluorescence detection.

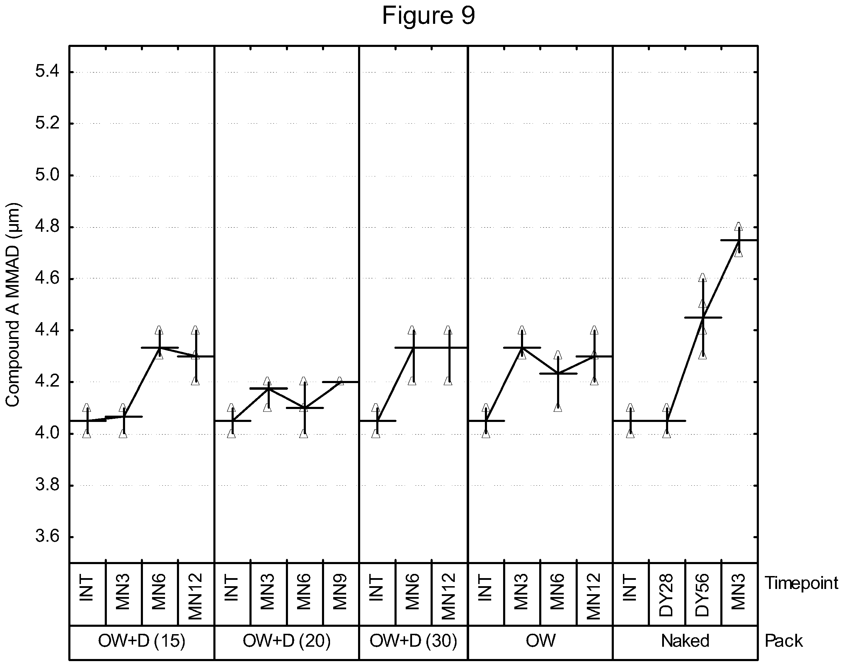

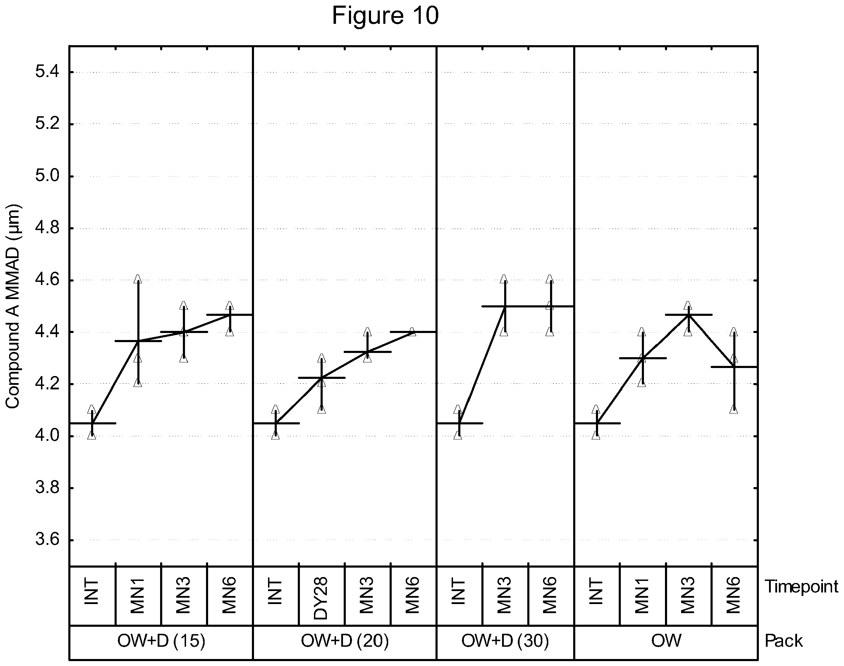

Figures 9-10 are illustrative of the changes in MMAD, in microns, as a function of both desiccant RH values and storage time. Figure 9 shows MMAD values for samples stored overwrapped (OW) and overwrapped with desiccant (OW+D) at 25°C and unprotected (naked) at 25 °C and 75%RH at select time intervals for compound A. Figure 10 shows MMAD values for samples stored overwrapped (OW) and overwrapped with desiccant (OW+D) at 40°C at select time intervals for compound A.

### Example 5

### Relationship of desiccant, humidity and temperature to inhalable formulation mass median aerodynamic diameter (MMAD)

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7%, 6.2% and 5.7% < than 4.5 microns by dry dispersion respectively), 25µg of Compound B, 1% magnesium stearate, and the balance to 12.5mg of inhalation grade lactose (lactose fines 4.5% <4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet (using foil laminate 1) or left unprotected (naked). Desiccant humidity values were 10% (8g Eq-pak®, 10% RH), 15% (8g Eq-pak®, 10% RH plus 8g Eq-pak®, 20% RH), 20% (8g Eq-pak®, 20% RH), 30% (8g Eq-pak®, 30% RH) and 40% (8g Eq-pak®, 40% RH). Overwrapped and desiccated devices were subsequently stored in standard stability chambers and storage conditions were 25°C and 60% RH and 40°C and 75% RH. Samples were analyzed for mass median aerodynamic diameter (MMAD) at time intervals up to 6 months for samples stored at 25°C and 60% RH and up to 7 months for samples stored at 40°C and 75% RH. MMAD was determined using cascade impaction with HPLC coupled to UV/visible and fluorescence detection.

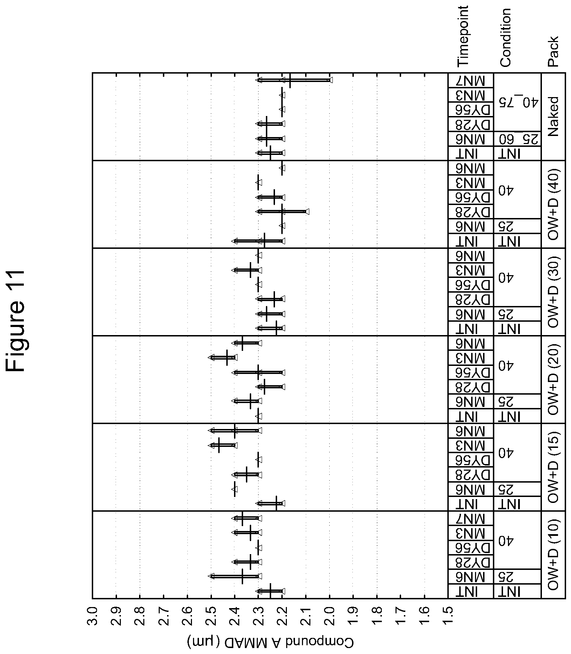

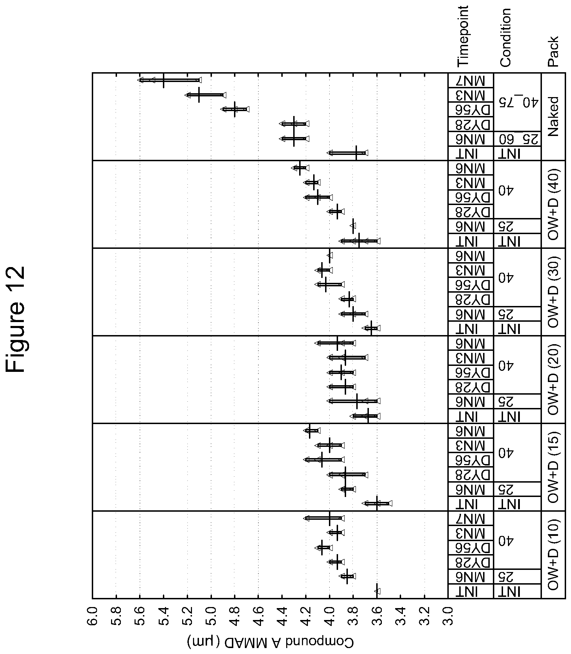

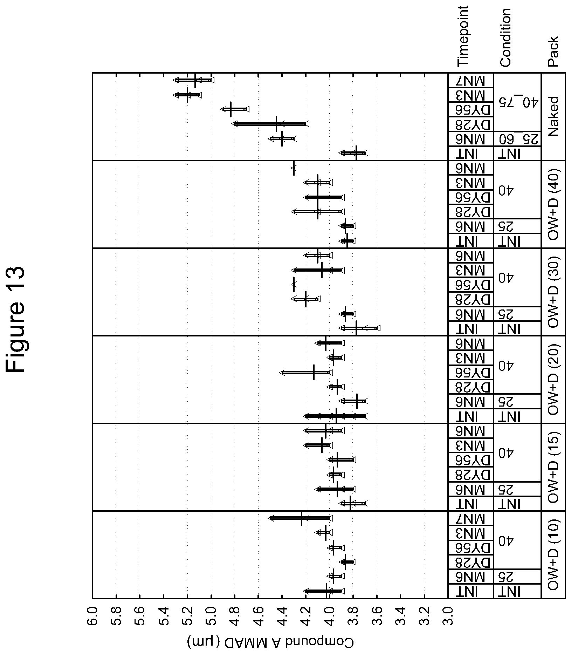

Figures 11-13 are illustrative of the changes in total for mass median aerodynamic diameter in microns as a function of both desiccant and storage conditions at select time intervals for the 50/25, 100/25, and 200/25 samples overwrapped with desiccant (OW+D) and unprotected. More specifically, Figures 11-13 show the changes in mass median aerodynamic diameter for blends 50/25, 100/25 and 200/25 for Compound A at 25°C/60% and 40°C/75% RH respectively.

### Example 6

### Relationship of desiccant, humidity and temperature to inhalable formulation geometric standard deviation (GSD)

Blends were manufactured and filled using standard equipment. The inhalation device contained two packs in peelable blister strip form, the first containing blend with 12.5 µg of compound B, 1% magnesium stearate, and the balance to 12.5mg of inhalation grade lactose (lactose fines 4.5% < than 4.5 microns by dry dispersion) and the second peelable blister strip containing 50 µg of compound A and the balance to 12.5mg of inhalation grade (lactose fines 6.5% < than 4.5 microns by dry dispersion) lactose. Samples were left unprotected, overwrapped or overwrapped with a desiccant packet, using foil laminate 2. Desiccant humidity values were 15% (8g Eq-pak®, 10% RH plus 8g Eq-pak®, 20% RH), 20% (8g Eq-pak®, 20% RH) and 30% (8g Eq-pak®, 30% RH). The RH inside the OW packs was around 45% RH. Overwrapped samples and samples overwrapped with desiccant were subsequently stored in standard stability chambers and storage conditions were 25°C and 60% RH and 40°C and 75% RH. Unprotected samples were stored in standard stability chambers and storage conditions were 25°C and 75% RH. Samples were analyzed for GSD initially and at time intervals up to 3 months for unprotected product stored at 25°C and 75%RH, up to 6 months for overwrapped and overwrapped with desiccant samples stored at 40°C and 75% RH and up to 9 or 12 months for overwrapped and overwrapped with desiccant samples stored at 25°C and 60% RH. GSD was determined using cascade impaction with HPLC coupled to UV/visible and fluorescence detection.

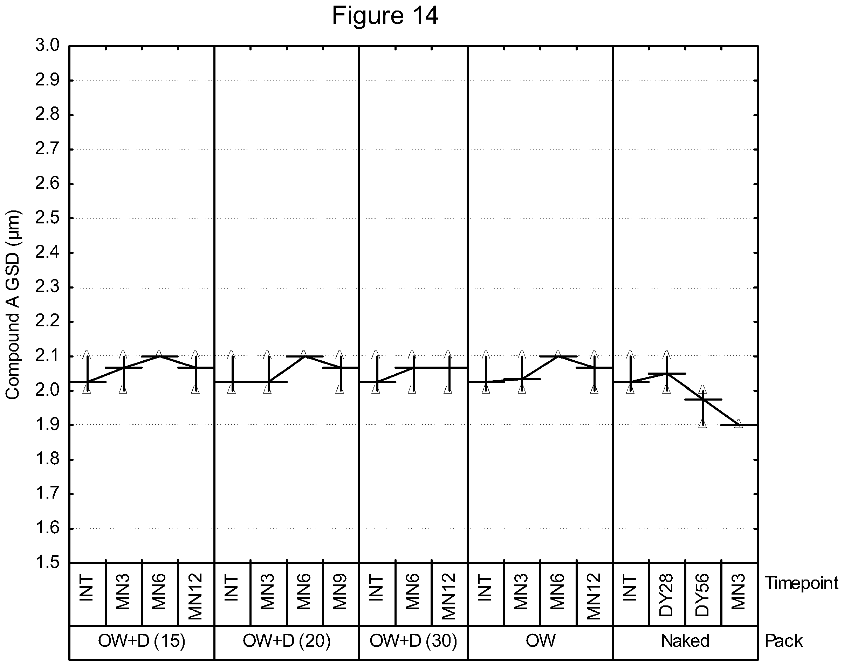

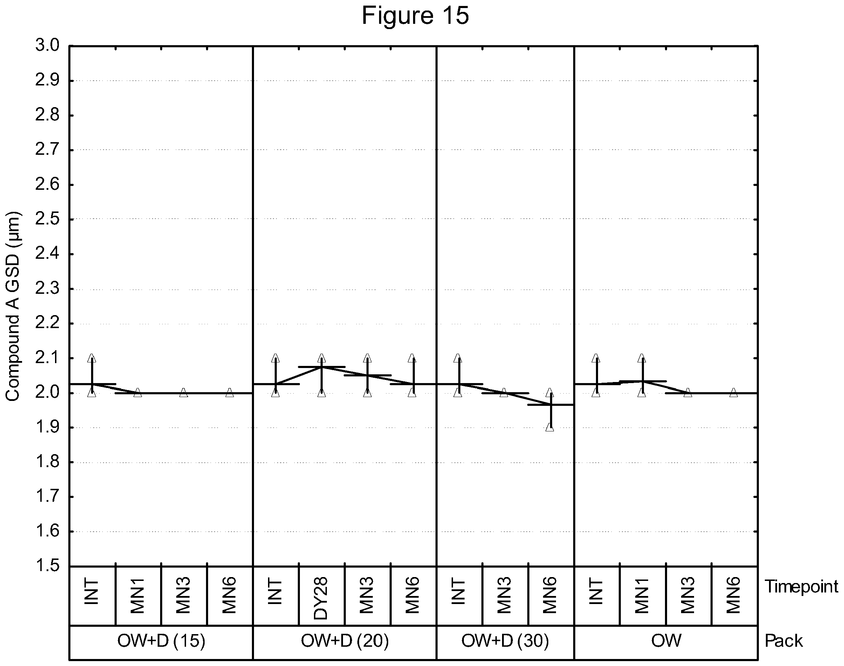

Figures 14-15 are illustrative of the changes in GSD, as a function of both desiccant RH values and storage time. Figure 14 shows GSD values for samples stored overwrapped (OW) and overwrapped with desiccant (OW+D) at 25°C and unprotected at 25 °C and 75%RH at select time intervals for compound A. Figure 15 shows GSD values for samples stored overwrapped (OW) and overwrapped with desiccant (OW+D) at 40°C at select time intervals for compound A.

### Example 7

### Relationship of desiccant, humidity and temperature to inhalable formulation geometric standard deviation (GSD)

Blends were manufactured and filled using standard equipment. Blends were packaged in strips as individual actives and in devices as dual active products (i.e., one strip of each active). The blends consisted of three levels of active ingredient, 50µg, 100µg and 200µg of Compound A and the balance to 12.5mg inhalation grade lactose (lactose fines 7%, 6.2% and 5.7% < than 4.5 microns by dry dispersion respectively), 25µg of Compound B, 1% magnesium stearate, and the balance to 12.5mg of inhalation grade lactose (lactose fines 4.5% <4.5 microns by dry dispersion). Final packaged device drug contents were 50µg/25µg, 100µg /25µg, and 200µg /25µg of Compound A and Compound B, respectively (henceforth referred to as 50/25, 100/25, and 200/25). Devices were overwrapped with a desiccant packet or left unprotected. Desiccant humidity values were 10% (8g Eq-pak®, 10% RH), 15% (8g Eq-pak®, 10% RH plus 8g Eq-pak®, 20% RH), 20% (8g Eq-pak®, 20% RH), 30% (8g Eq-pak®, 30% RH) and 40% (8g Eq-pak®, 40% RH). Overwrapped (using foil laminate 1) and Desiccated devices were subsequently stored in standard stability chambers alongside unprotected devices and storage conditions were 25°C and 60% RH and 40°C and 75% RH. Samples were analyzed for geometric standard deviation (GSD) at time intervals up to 6 months for samples stored at 25°C and 60% RH and up to 7 months for samples stored at 40°C and 75%. GSD was determined using cascade impaction with HPLC coupled to UV/visible and fluorescence detection.

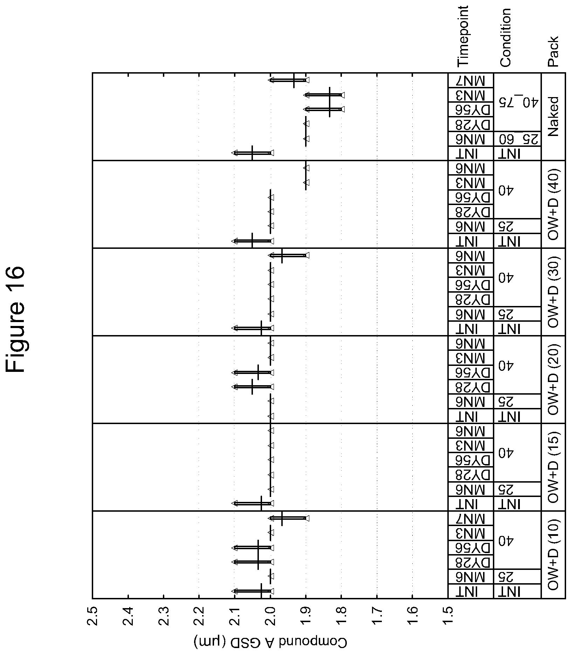

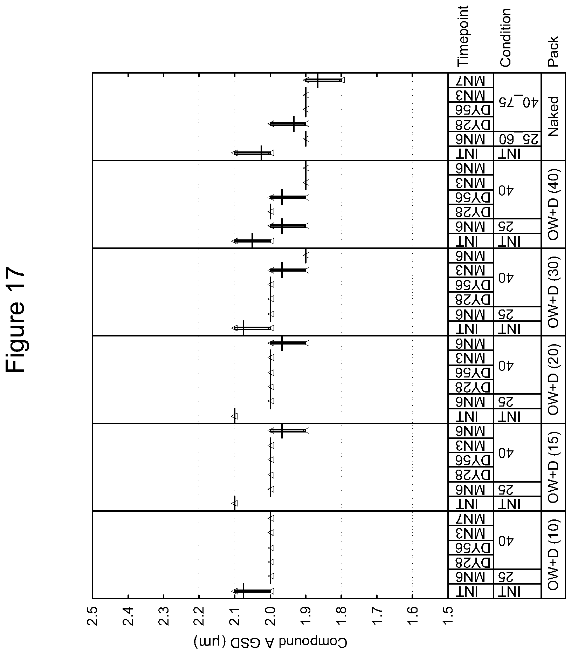

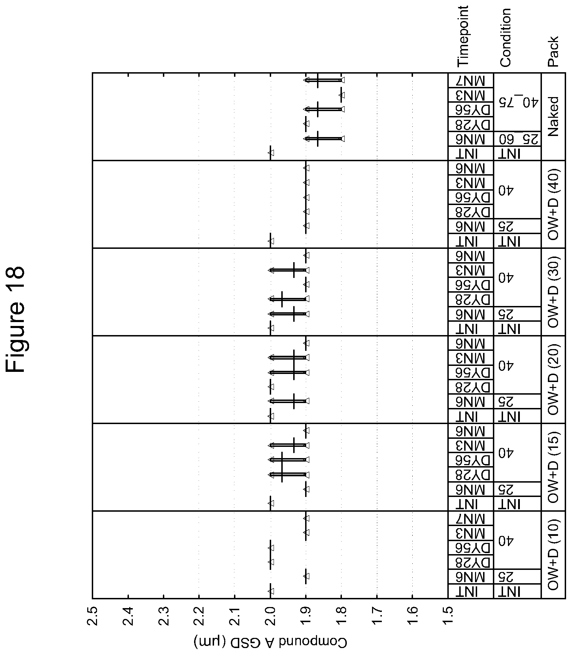

Figures 16-18 are illustrative of the changes in Geometric Standard Deviation as a function of both desiccant and storage conditions at select time intervals for the 50/25, 100/25, and 200/25 samples overwrapped with desiccant (OW+D) and unprotected, and more specifically show the changes in geometric standard deviation for blends 50/25, 100/25 and 200/25 for Compound A at 25°C/60% and 40°C/75% RH respectively.

The application of which this description and claims form part may be used as a basis for priority in respect of any subsequent application. The claims of such subsequent application may be directed to any feature or combination of features described therein. They may take the form of product, method or use claims and may include, by way of example and without limitation, one or more of the following claims.

## Claims

1. A drug product comprising:
a dry powder inhalation device having a pharmaceutical composition present therein, said pharmaceutical composition comprising a compound which is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof; a hygroscopic material,
and a package which encompasses the dry powder inhalation device and the hygroscopic material defining an enclosed volume therein, wherein said hygroscopic material is in a state of partial hydration and wherein the enclosed volume exhibits a Relative Humidity of from 20% to 40%.

2. The drug product according to Claim1, wherein said compound is (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate.

3. The drug product according to Claim 1, wherein said pharmaceutical composition comprises at least one excipient.

4. The drug product according to Claim 3, wherein said at least one excipient is lactose.

5. The drug product according to Claim 1, wherein said hygroscopic material comprises a desiccant.

6. The drug product according to Claim 1, wherein said hygroscopic material comprises silica gel.

7. The drug product according to Claim 6 wherein the silica gel is present in an enclosure.

8. The drug product according to Claim 7, wherein the silica gel is present in a sachet.

9. The drug product according to any of Claims 1 to 8, wherein the sachet is loose within the drug product.

10. The drug product according to any of Claims 1 to 9, wherein the sachet is fixed with respect to the inhalation device.

11. The drug product according to Claim 1, wherein the dry powder inhalation device is a unit-dose device.

12. The drug product according to Claim 1, wherein the dry powder inhalation device is a multi-dose device.

13. A drug product as claimed in claim 1 for use in treating a respiratory disorder.

14. A process of producing a drug product comprising:
subjecting a hygroscopic material to moisture exposure sufficient to attain a predetermined relative humidity;
combining the hygroscopic material with an inhalation device containing a pharmaceutical composition therein, wherein the pharmaceutical composition comprises (6α,11β,16α,17α)-6,9-difluoro-17-{[(fluoromethyl)thio]carbonyl}-11-hydroxy-16-methyl-3-oxoandrosta-1,4-dien-17-yl 2- furancarboxylate or a solvate thereof;
enclosing the dry powder inhalation device and hygroscopic material within a package to define an enclosed volume therein forming a drug product and wherein the hydration level of the hygroscopic material is such that enclosed volume has a Relative Humidity of from 20% to 40%.

15. A drug product produced according to the process of Claim 14.

## Patentansprüche

1. Arzneimittelprodukt, umfassend:
eine Trockenpulver-Inhalationsvorrichtung, die darin eine pharmazeutische Zusammensetzung aufweist, wobei die pharmazeutische Zusammensetzung eine Verbindung, die (6α,11β,16α,17α)-6,9-Difluor-17-{[(fluormethyl)thio]carbonyl}-11-hydroxy-16-methyl-3-oxoandrosta-1,4-dien-17-yl-2-furancarboxylat ist, oder ein Solvat davon umfasst;
ein hygroskopisches Material; und
eine Verpackung, die die Trockenpulver-Inhalationsvorrichtung und das hygroskopische Material, das ein darin eingeschlossenes Volumen definiert, umfasst;
wobei sich das hygroskopische Material in einem Zustand teilweiser Hydratation befindet und wobei das eingeschlossene Volumen eine relative Feuchtigkeit von 20 bis 40 % aufweist.

2. Arzneimittelprodukt gemäss Anspruch 1, wobei die Verbindung (6α,11β,16α,17α)-6,9-Difluor-17-{[(fluormethyl)thio]carbonyl}-11-hydroxy-16-methyl-3-oxoandrosta-1,4-dien-17-yl-2-furancarboxylat ist.

3. Arzneimittelprodukt gemäss Anspruch 1, wobei die pharmazeutische Zusammensetzung zumindest einen Hilfsstoff umfasst.

4. Arzneimittelprodukt gemäss Anspruch 3, wobei der zumindest eine Hilfsstoff Lactose ist.

5. Arzneimittelprodukt gemäss Anspruch 1, wobei das hygroskopische Material ein Trockenmittel ist.

6. Arzneimittelprodukt gemäss Anspruch 1, wobei das hygroskopische Material Silicagel umfasst.

7. Arzneimittelprodukt gemäss Anspruch 6, wobei das Silicagel in einer Umhüllung vorhanden ist.

8. Arzneimittelprodukt gemäss Anspruch 7, wobei das Silicagel in einem Sachet vorhanden ist.

9. Arzneimittelprodukt gemäss einem der Ansprüche 1 bis 8, wobei das Sachet lose innerhalb des Arzneimittelprodukts vorliegt.

10. Arzneimittelprodukt gemäss einem der Ansprüche 1 bis 9, wobei das Sachet in bezug auf die Inhalationsvorrichtung fixiert ist.

11. Arzneimittelprodukt gemäss Anspruch 1, wobei die Trockenpulver-Inhalationsvorrichtung eine Einzeldosis-Vorrichtung ist.

12. Arzneimittelprodukt gemäss Anspruch 1, wobei die Trockenpulver-Inhalationsvorrichtung eine Mehrfachdosis-Vorrichtung ist.

13. Arzneimittelprodukt gemäss Anspruch 1 zur Verwendung bei der Behandlung einer Atmungsstörung.

14. Verfahren zur Herstellung eines Arzneimittelprodukts, umfassend:
Unterziehen eines hygroskopischen Materials einer Feuchtigkeitseinwirkung, die ausreicht, um eine vorbestimmte relative Feuchtigkeit zu erreichen;
Kombinieren des hygroskopischen Materials mit einer Inhalationsvorrichtung, die eine pharmazeutische Zusammensetzung darin enthält, wobei die pharmazeutische Zusammensetzung (6α,11β,16α,17α)-6,9-Difluor-17-{[(fluormethyl)thio]carbonyl}-11-hydroxy-16-methyl-3-oxoandrosta-1,4-dien-17-yl-2-furancarboxylat oder ein Solvat davon umfasst;
Einschliessen der Trockenpulver-Inhalationsvorrichtung und des hygroskopischen Materials in einer Verpackung, um darin ein eingeschlossenes Volumen zu definieren, das ein Arzneimittelprodukt bildet, und wobei der Hydratationsgrad des hygroskopischen Materials derart ist, dass das eingeschlossene Volumen eine relative Feuchtigkeit von 20 bis 40 % aufweist.

15. Arzneimittelprodukt, hergestellt nach dem Verfahren gemäss Anspruch 14.

## Revendications

1. Produit médicamenteux comprenant :
un dispositif d'inhalation de poudre sèche ayant une composition pharmaceutique qui y est présente, ladite composition pharmaceutique comprenant un composé qui est le 2-furancarboxylate de (6α,11β,16α,17α)-6,9-difluoro-17-{[(fluorométhyl)thio]carbonyl}-11-hydroxy-16-méthyl-3-oxoandrosta-1,4-dién-17-yle ou un solvate de celui-ci ; un matériau hygroscopique et un conditionnement qui enserre le dispositif d'inhalation de poudre sèche et le matériau hygroscopique y définissant un volume fermé, dans lequel ledit matériau hygroscopique est dans un étant d'hydratation partielle et dans lequel le volume fermé présente une humidité relative de 20 % à 40 %.

2. Produit médicamenteux selon la revendication 1, dans lequel ledit composé est le 2-furancarboxylate de (6α,11β,16α,17α)-6,9-difluoro-17-{[(fluorométhyl)thio]carbonyl}-11-hydroxy-16-méthyl-3-oxoandrosta-1,4-dién-17-yle.

3. Produit médicamenteux selon la revendication 1, dans lequel ladite composition pharmaceutique comprend au moins un excipient.

4. Produit médicamenteux selon la revendication 3, dans lequel ledit au moins un excipient est le lactose.

5. Produit médicamenteux selon la revendication 1, dans lequel ledit matériau hygroscopique comprend un dessiccant.

6. Produit médicamenteux selon la revendication 1, dans lequel ledit matériau hygroscopique comprend un gel de silice.

7. Produit médicamenteux selon la revendication 6, dans lequel le gel de silice est présent dans une enceinte.

8. Produit médicamenteux selon la revendication 7, dans lequel le gel de silice est présent dans un sachet.

9. Produit médicamenteux selon l'une quelconque des revendications 1 à 8, dans lequel le sachet est libre dans le produit médicamenteux.

10. Produit médicamenteux selon l'une quelconque des revendications 1 à 9, dans lequel le sachet est fixé par rapport au dispositif d'inhalation.

11. Produit médicamenteux selon la revendication 1, dans lequel le dispositif d'inhalation de poudre sèche est un dispositif à dose unique.

12. Produit médicamenteux selon la revendication 1, dans lequel le dispositif d'inhalation de poudre sèche est un dispositif à doses multiples.

13. Produit médicamenteux selon la revendication 1 pour utilisation dans le traitement d'un trouble respiratoire.

14. Procédé de production d'un produit médicamenteux comprenant les étapes consistant à :
soumettre un matériau hygroscopique à une exposition à de l'humidité suffisamment pour atteindre une humidité relative prédéterminée ;
combiner le matériau hygroscopique avec un dispositif d'inhalation contenant une composition pharmaceutique, dans lequel la composition pharmaceutique comprend le 2-furancarboxylate de (6α,11β,16α,17α)-6,9-difluoro-17-{[(fluorométhyl)thio]carbonyl}-11-hydroxy-16-méthyl-3-oxoandrosta-1,4-dién-17-yle ou un solvate de celui-ci ;
enfermer le dispositif d'inhalation de poudre sèche et le matériau hygroscopique dans un conditionnement pour y définir un volume fermé formant un produit médicamenteux et dans lequel le niveau d'hydratation du matériau hygroscopique est tel que le volume fermé ait une humidité relative de 20 % à 40 %.

15. Produit médicamenteux selon le procédé de la revendication 14.
